# EUROPEAN PATENT SPECIFICATION

(11) **EP 1 743 937 B1**
(45) Date of publication and mention of the grant of the patent: **23.05.2012**
(21) Application number: 06014387.2
(22) Date of filing: 11.07.2006
(51) Int. Cl.: C12N 11/04, C12N 11/08

(54) **Microorganism-entrapping immobilization pellets and process for producing the same**
Immobilisierungs-Tabletten für den Einschluss von Mikroorganismen und Prozess zur Herstellung derselben.
Granules immobilisants enfermant des microorganismes et le procédé pour produire la même chose

(30) Priority: 13.07.2005 JP 2005204453
(43) Date of publication of application: 17.01.2007
(73) Proprietor: Hitachi Plant Technologies, Ltd., Tokyo (JP)
(72) Inventor: Aoyama, Koutarou, Chiyoda-ku Tokyo (JP); Abe, Naoki, Chiyoda-ku Tokyo (JP); Sumino, Tatsuo, Chiyoda-ku Tokyo (JP)
(74) Representative: Grünecker, Kinkeldey, Stockmair & Schwanhäusser

(56) References cited:
- EP-A- 0 186 125
- WO-A-97/12965
- PATENT ABSTRACTS OF JAPAN vol. 1998, no. 03, 27 February 1998 (1998-02-27) & JP 09 296066 A (OGURA BOEKI KK; AKESE HIROHIKO), 18 November 1997 (1997-11-18) & JP 09 296066 A (OGURA BOEKI KK; AKESE HIROHIKO) 18 November 1997 (1997-11-18)
- PATENT ABSTRACTS OF JAPAN vol. 2000, no. 20, 10 July 2001 (2001-07-10) & JP 2001 064427 A (NAGANO NOVA FORM KK), 13 March 2001 (2001-03-13) & JP 2001 064427 A (NAGANO NOVA FORM KK) 13 March 2001 (2001-03-13)

## Description

### BACKGROUND OF THE INVENTION

### Field of the Invention

The present invention relates to a process for producing entrapping immobilization pellets. More particularly, the present invention relates to improvement of pellet strength of entrapping immobilization pellets used for treatment of nitrogen and organic substances in rivers, lakes, sewage, or industrial wastewater.

### Description of the Related Art

Conventionally, as a process for treating wastewater such as sewage, industrial wastewater or agricultural wastewater, a biological process has been widely used, because the process involves a low cost as compared with a physicochemical process. Typical examples include an activated sludge process often used for sewage. However, since slowly growing microorganisms such as nitrifying bacteria in activated sludge which are highly involved in removing organic substances or nitrogen in wastewater flow out of a reaction tank without sufficient growth, the reaction speed is significantly decreased particularly when the water temperature is low, resulting in deterioration of water quality. For this reason, various processes for retaining a high concentration of nitrifying bacteria or the like in a reaction tank in a stable manner have been studied, and techniques for immobilizing microorganisms in pellets have been practically used as such processes. Such microorganism immobilization processes are roughly classified into microorganism attachment processes and microorganism entrapping immobilization processes. Microorganism attachment processes comprise packing microorganisms in activated sludge or the like with cylindrical or cubic attachment pellets to naturally attach the microorganisms to the surface of the pellets. Microorganism entrapping immobilization processes comprise retaining microorganisms in activated sludge or the like in a synthetic polymer compound such as polyvinyl alcohol, acrylamide, or polyethylene glycol, or a naturally occurring material such as agar or alginic acid. As is clear from comparison of these two types, microorganism entrapping immobilization processes are excellent in activity rising and stability as compared with microorganism attachment processes utilizing naturally attached microorganisms.

For example, Japanese Patent Application Laid-Open No. 11-18765 discloses microorganism entrapping immobilization pellets that contain a magnetic body and can control flowing or recovery of the entrapping immobilization pellets by magnetic force. It is assumed that wastewater can be effectively treated with the entrapping immobilization pellets.

Japanese Patent Application Laid-Open No. 2003-235554 discloses entrapping immobilization pellets in which activated carbon and microorganisms are immobilized at the same time to retain a large number of bacterial cells as compared with conventional pellets. Also disclosed is a process for producing the entrapping immobilization pellets comprising mixing an immobilizing material with microorganisms and entrapping and immobilizing the microorganisms by polymerization reaction or freezing and defrosting treatment. It is assumed that this process can provide entrapping immobilization pellets having a decreased amount of microorganisms deactivated or killed during polymerization and having practical pellet strength.

However, microorganisms such as nitrifying bacteria are often killed during or after immobilization by the direct effect of an immobilizing material or a chemical such as a polymerization initiator on microbial activity or the effect of pH change, temperature change, or the like in the pellets during immobilization. This causes problems in that nitrification activity of entrapping immobilization pellets varies depending on the production lots, or it must take a long acclimatization period to enhance nitrification activity of entrapping immobilization pellets, for example.

Accordingly, in order to produce entrapping immobilization pellets having stable nitrification activity, it is important to immobilize microorganisms without affecting the microorganisms. Specifically, the effect on microorganisms can be considerably reduced by reducing the amount of an immobilizing material or a chemical such as a polymerization initiator.

However, when the amount of an immobilizing material or a chemical such as a polymerization initiator is reduced, entrapping immobilization pellets have decreased strength, making the life of the pellets shorter in wastewater treatment. For example, entrapping immobilization pellets of Japanese Patent Application Laid-Open No. 2003-235554 have high activity because of activated carbon added, but have reduced pellet strength, disadvantageously.

The present invention has been achieved in view of such circumstances. An object of the present invention is to provide entrapping immobilization pellets which have high pellet strength and retain high and stable microbial activity (nitrification activity) even if a low concentration of a material involved in immobilization (such as an immobilizing material or polymerization initiator) is used, and a process for producing the same.

### SUMMARY OF THE INVENTION

To attain the aforementioned object, according to a first aspect of the present invention, there is provided a process for producing entrapping immobilization pellets in which microorganisms are entrapped and immobilized in an immobilizing material, the entrapping immobilization pellets comprising a filler having a plate-like and/or needle-like crystal structure in the immobilizing material.

The filler used herein refers to relatively inactive particles or powdery substance added to a material such as a resin, rubber or paint to improve the strength and functions and reduce the cost, and is called a packing agent or bulking agent. The entrapping immobilization pellets of the present invention comprise a filler having a plate-like and/or needle-like crystal structure in an immobilizing material. The filler is dispersed in the immobilizing material (aqueous gel) in a network or fibrous shape, unlike a spherical filler, making fluidity of the aqueous gel decreased. The plate-like or needle-like filler easily enters the polymer gel network. Accordingly, the plate-like or needle-like filler forms a rigid and substantially networked structure, and thus reinforces and immobilizes the aqueous gel and increases the pellet strength. Moreover, since the amount of the immobilizing material or a chemical such as a polymerization initiator can be reduced, a decrease in microbial activity by the chemical can be prevented. In addition, since the entrapping immobilization pellets using the plate-like or needle-like filler are rigid, the pellets can be cut into pieces with a uniform shape during production, and the produced entrapping immobilization pellets can have high shape accuracy and stable quality.

In the first aspect, the immobilizing material used may be a high-molecular-weight monomer, a prepolymer, an oligomer, or the like. The filler may be an inorganic filler or an organic filler, and is particularly preferably a plate-like filler to increase the pellet strength. Preferable examples include talc, plate-like alumina, synthetic mica, kaolinite, Sillitin, and Aktisil. The needle-like filler also refers to a fibrous or rod-like filler. The plate-like or needle-like filler may be added in combination with a spherical filler.

According to a second aspect of the present invention, there is provided the entrapping immobilization pellets according to the first aspect, wherein the plate-like and/or needle-like filler is an inorganic filler.

In the second aspect, the type of the filler is specified. The filler of the second aspect is particularly effective for increasing the pellet strength. Specific preferable examples include talc, plate-like alumina, synthetic mica, kaolinite, Sillitin, Aktisil, a mica powder, a zinc oxide whisker, wollastonite, potassium titanate, a magnesium sulfate whisker, a calcium silicate whisker, mica, a graphite powder, and a carbon fiber.

According to a third aspect of the present invention, there is provided the entrapping immobilization pellets according to the first or second aspect, wherein the plate-like and/or needle-like filler has an average particle size of 12 µm or less.

When the filler has an average particle size defined in the third aspect, the interface area between the immobilizing material (aqueous gel) and the filler can be increased, and dispersibility of the filler can be improved. Therefore, even a small amount of the immobilizing material is added, entrapping immobilization pellets having uniform and high pellet strength can be produced. The average particle size is more preferably 10 µm or less. The average particle size is a particle size of a spherical filler having the same volume as in a plate-like or needle-like filler.

According to a fourth aspect of the present invention, there is provided the entrapping immobilization pellets according to any one of the first to third aspects, wherein the mass ratio F/P of the mass of the plate-like and/or needle-like filler F to the mass of the immobilizing material P is 0.01 to 5.

According to the fourth aspect, even if the amount of the immobilizing material is decreased, entrapping immobilization pellets having high pellet strength and microbial activity can be produced. The mass ratio F/P is more preferably 0.04 to 3.5.

According to a fifth aspect of the present invention, there is provided the entrapping immobilization pellets according to any one of the first to fourth aspects, wherein the concentration of the plate-like and/or needle-like filler is 0.1 to 20 mass% based on the entrapping immobilization pellets, and the concentration of the immobilizing material is 2 to 15 mass% based on the entrapping immobilization pellets.

According to the fifth aspect, even a low concentration of the immobilizing material that does not reduce microbial activity can retain high pellet strength. Thus, variation of pellet activity according to the production lots can be eliminated. The concentration of the immobilizing material is more preferably 3 to 10 mass% based on the entrapping immobilization pellets.

To attain the aforementioned object, according to a sixth aspect of the present invention, there is provided a process for producing entrapping immobilization pellets in which microorganisms are entrapped and immobilized in an immobilizing material, the process comprising mixing the immobilizing material with a filler having a plate-like and/or needle-like crystal structure, and then mixing the mixture with the microorganisms to form a gel.

In the sixth aspect, the present invention is applied to a production process. According to the sixth aspect, an immobilizing material is first mixed with a filler having a plate-like and/or needle-like crystal structure. Thus, generation of balls as a result of mixing the filler with microorganisms can be prevented. Therefore, the filler and the microorganisms can be homogeneously mixed and dispersed, and entrapping immobilization pellets having high pellet strength and high microbial activity can be produced. Further, variation in product quality can be reduced, and stability of quality can be improved. In the sixth aspect, the filler may be an inorganic filler or an organic filler, and is particularly preferably a plate-like filler to increase the pellet strength. The needle-like filler also refers to a fibrous or rod-like filler.

According to a seventh aspect of the present invention, there is provided the process for producing entrapping immobilization pellets according to the sixth aspect, wherein the plate-like and/or needle-like filler is an inorganic filler.

When an inorganic filler is first mixed with microorganisms in activated sludge or the like and then the mixture is mixed with an immobilizing material, balls are easily formed, and the inorganic filler and the microorganisms cannot be homogeneously dispersed. According to the seventh aspect, since an immobilizing material is first mixed with an inorganic filler, the inorganic filler and microorganisms can be homogeneously dispersed. Therefore, entrapping immobilization pellets having high pellet strength and high microbial activity can be obtained. Variation of pellet activity according to the production lots can also be eliminated.

As described above, the present invention can produce entrapping immobilization pellets which have high pellet strength and retain high and stable microbial activity (nitrification activity) even if a low concentration of a material involved in immobilization (such as an immobilizing material or polymerization initiator) is used.

### BRIEF DESCRIPTION OF THE DRAWINGS

Fig. 1 is a flow chart describing the process for producing entrapping immobilization pellets to which the present invention is applied;
Fig. 2 is a graph showing the relation between the shape and amount added of the filler and the pellet strength in Example 2 of the present invention;
Fig. 3 is a graph showing the relation between the average particle size of the filler and the pellet strength in Example 3 of the present invention; and
Fig. 4 is a graph showing evaluation of nitrification performance of the pellets in Example 4 of the present invention.

### DETAILED DESCRIPTION OF THE PREFERRED EMBODIMENTS

A preferred embodiment of the entrapping immobilization pellets and the process for producing the same of the present invention will be described in detail below with reference to the accompanying drawings.

In the entrapping immobilization pellets of the present invention, microorganisms are entrapped and immobilized in an immobilizing material containing a plate-like and/or needle-like filler.

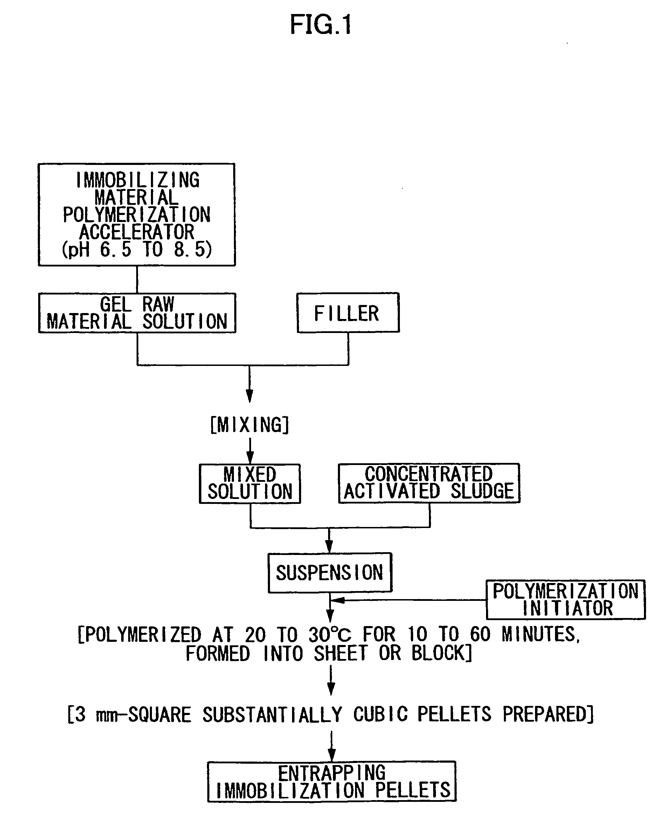

Fig. 1 is a flow chart showing an example of the process for producing entrapping immobilization pellets of the present invention, in which 3 mm-square substantially cubic pellets are produced. As shown in Fig. 1, an immobilizing material is mixed with a polymerization accelerator to prepare a gel raw material solution having pH adjusted to around the neutrality (6.5 to 8.5). Next, the gel raw material solution is mixed with a plate-like and/or needle-like filler to prepare a mixed solution. Then, concentrated activated sludge is suspended in the mixed solution to prepare a suspension. A polymerization initiator is added to the suspension, and the mixture is polymerized. In this case, the mixture is gelled and formed into a sheet or block by polymerization. Here, gelation is carried out at a polymerization temperature of 15 to 40°C, and preferably 20 to 30°C, for a polymerization time of 5 to 60 minutes, and preferably 10 to 60 minutes. Next, the gelled sheet or block is cut into about 3 mm-square substantially cubic pellets to produce entrapping immobilization pellets.

In the entrapping immobilization pellets of the present invention thus produced, the plate-like or needle-like filler is dispersed in the immobilizing material in a network or fibrous shape, and easily enters the aqueous gel network. Therefore, the filler can restrict a flow of the aqueous gel to a large extent and can immobilize the aqueous gel in a small amount added, as compared with the case of a spherical filler. Further, since the amount of the immobilizing material or a chemical such as the polymerization initiator can be reduced, a decrease in microbial activity can be prevented. In addition, since the aqueous gel can have increased viscosity to make the microorganisms highly homogeneously dispersed in the whole immobilizing material while reducing the risk of sedimentation of the microorganisms, efficiency in utilizing microbial activity can also be improved.

In the process for producing entrapping immobilization pellets, since the immobilizing material is first mixed with the plate-like and/or needle-like filler, the filler can be homogeneously dispersed in the immobilizing material. Therefore, balls are not generated when the mixture of the immobilizing material and the filler is mixed with the microorganisms, and entrapping immobilization pellets with stable quality can be produced.

Suitable examples of the microorganisms used in the present embodiment include nitrifying bacteria or complex microorganisms comprising nitrifying bacteria, denitrifying bacteria, and anaerobic ammonium oxidizing bacteria for the purpose of removing nitrogen; and microorganisms that can decompose specific toxic chemical substances such as dioxins (pure microorganisms such as water-bloom decomposing bacteria, PCB decomposing bacteria, dioxin decomposing bacteria, and environmental hormone decomposing bacteria, for example). The microorganisms refer not only to microorganisms concentrated and separated by culturing or the like, but also to substances containing various microorganisms such as activated sludge in sewage treatment plants, sludge in lakes, rivers, or sea, and soil.

As gelation treatment, a polymerization method comprising gelling the immobilizing material by polymerization reaction is generally used. However, when polyvinyl alcohol (PVA) is used as the immobilizing material, gelation treatment may be carried out by a PVA freezing method comprising mixing PVA with microorganisms and then repeating freezing and defrosting to carry out gelation reaction; or by a PVA-boric acid method comprising mixing PVA with microorganisms and then mixing the mixture with boric acid to carry out gelation reaction.

Preferable examples of the plate-like or needle-like filler used in the present embodiment include talc, plate-like alumina, kaolinite, Sillitin, Aktisil, a mica powder, a zinc oxide whisker, wollastonite, potassium titanate, a magnesium sulfate whisker, a calcium silicate whisker, mica, synthetic mica, a graphite powder, and a carbon fiber.

Further preferable examples include plate-like or needle-like fillers made of fly ash, an activated carbon powder, calcium carbonate, calcium sulfate, calcium sulfite, magnesium hydroxide, aluminum hydroxide, antimony oxide, zinc stannate, titanium oxide, zinc oxide, a silica powder, glass beads, diatomite, calcium silicate, attapulgite, asbestos, carbon black, acetylene black, furnace black, white carbon, pyrophyllite clay, silica, cotton, polyester, nylon, a graphite powder, silicon nitride, molybdenum disulfide, iron oxide, basic magnesium carbonate, hydrotalcite, alumina, zirconium oxide, bentonite, zeolite, kaolin clay, sericite, zirconium silicate, barium sulfate, barium carbonate, barium titanate, zinc oxide, kaolin, sepiolite, smectite, vermiculite, ampholytic polyacrylamide, pyrophyllite, cationic polyacrylamide, anionic polyacrylamide, agar, gellan gum, chitin, chitosan, cellulose, collagen, polyamino acid, gelatin, and casein. Further, the plate-like or needle-like filler may be used in combination with a spherical filler made of any of the substances referred to in the preceding paragraph.

Examples of the immobilizing material used in the present embodiment include, a monomer, a prepolymer, and an oligomer, but the immobilizing material is not specifically limited. For example, polyacrylamide, polyvinyl alcohol, polyethylene glycol, sodium alginate, carrageenan, or agar can be used. Examples of the immobilizing agent prepolymer that can be used include the following compounds:
monomethacrylates such as polyethylene glycol monomethacrylate, polyprene glycol monomethacrylate, polypropylene glycol monomethacrylate, methoxydiethylene glycol methacrylate, methoxypolyethylene glycol methacrylate, methacryloyloxyethyl hydrogen phthalate, methacryloyloxyethyl hydrogen succinate, 3-chloro-2-hydroxypropyl methacrylate, stearyl methacrylate, 2-hydroxy methacrylate, and ethyl methacrylate;
monoacrylates such as 2-hydroxyethyl acrylate, 2-hydroxypropyl acrylate, isobutyl acrylate, t-butyl acrylate, isooctyl acrylate, lauryl acrylate, stearyl acrylate, isobornyl acrylate, cyclohexyl acrylate, methoxytriethylene glycol acrylate, 2-ethoxyethyl acrylate, tetrahydrofurfuryl acrylate, phenoxyethyl acrylate, nonylphenoxypolyethylene glycol acrylate, nonylphenoxypolypropylene glycol acrylate, silicon-modified acrylate, polypropylene glycol monoacrylate, phenoxyethyl acrylate, phenoxydiethylene glycol acrylate, phenoxypolyethylene glycol acrylate, methoxypolyethylene glycol acrylate, acryloyloxyethyl hydrogen succinate, and lauryl acrylate;
dimethacrylates such as 1,3-butylene glycol dimethacrylate, 1,4-butanediol dimethacrylate, ethylene glycol dimethacrylate, diethylene glycol dimethacrylate, triethylene glycol dimethacrylate, polyethylene glycol dimethacrylate, butylene glycol dimethacrylate, hexanediol dimethacrylate, neopentyl glycol dimethacrylate, polyprene glycol dimethacrylate, 2-hydroxy-1,3-dimethacryloxypropane, 2,2-bis-4-methacryloxyethoxyphenylpropane, 3,2-bis-4-methacryloxydiethoxyphenylpropane, and 2,2-bis-4-methacryloxypolyethoxyphenylpropane;
diacrylates such as ethoxylated neopentyl glycol diacrylate, polyethylene glycol diacrylate, 1,6-hexanediol diacrylate, neopentyl glycol diacrylate, tripropylene glycol diacrylate, polypropylene glycol diacrylate, 2,2-bis-4-acryloxyethoxyphenylpropane, 2-hydroxy-1-acryloxy-3-methacryloxypropane;
trimethacrylates such as trimethylolpropane trimethacrylate;
triacrylates such as trimethylolpropane triacrylate, pentaerythritol triacrylate, trimethylolpropane EO-added triacrylate, glycerol PO-added triacrylate, and ethoxylated trimethylolpropane triacrylate;
tetraacrylates such as pentaerythritol tetraacrylate, ethoxylated pentaerythritol tetraacrylate, propoxylated pentaerythritol tetraacrylate, and ditrimethylolpropane tetraacrylate;
urethane acrylates such as urethane acrylate, urethane dimethyl acrylate, and urethane trimethyl acrylate; and
other compounds such as acrylamide, acrylic acid, and dimethylacrylamide.

The pellet polymerization in the present invention is most appropriately radical polymerization using potassium persulfate, but may be polymerization using ultraviolet rays or electron beams or redox polymerization. In polymerization using potassium persulfate, potassium persulfate is preferably added in an amount of 0.001 to 0.25 mass%, and an amine polymerization accelerator is preferably added in an amount of 0.01 to 0.5 mass%. As the amine polymerization accelerator, β-dimethylaminopropionitrile, N,N,N',N'-tetramethylethylenediamine, or the like can be preferably used.

In the present embodiment, a plate-like filler (talc) is mainly added. However, a combination of a plate-like filler, needle-like filler and spherical filler having different average particle sizes can also be used. Various fillers may be surface treated for controlling surface properties such as wettability with an immobilizing material and interface area to more efficiently use the fillers.

As described above, when the present invention is applied, microorganisms can be entrapped and immobilized in even a small amount of an immobilizing material with high pellet strength. Accordingly, a decrease in microbial activity by an immobilizing material or a chemical such as a polymerization initiator can be prevented, and entrapping immobilization pellets retaining high pellet strength and microbial activity which are durable even in long-term operation can be obtained. Further, variation in product quality according to the lots can be reduced, and stability of quality can be improved. In addition, since viscosity of the aqueous gel can be improved by addition of a plate-like and/or needle-like filler, microorganisms can be highly homogeneously dispersed in the whole immobilizing material while reducing the risk of sedimentation of the microorganisms. Thus, efficiency in utilizing microbial activity can also be improved.

### Examples

Examples of the present invention will be described below. However, the present invention is not limited to the examples.

First, the relation between the shape, amount, and average particle size of a filler added to an immobilizing material and the strength of entrapping immobilization pellets (hereinafter referred to as pellets) was examined. Next, continuous nitrification performance of the entrapping immobilization pellets of the present embodiment was evaluated. As the pellet materials, those of Fig. 1 were used. The pellet strength was measured using a rheometer as a compression force per unit area when compressing entrapping immobilization pellets with a certain force to break the pellets (meaning that, when the pellet strength is 7 kgf/cm² (68.6 N/cm²), the pellets are broken down by applying pressure above the compression force).

**Table 1**

| Composition | Composition |
|---|---|
| Activated sludge | 3 mass% |
| Number of nitrifying bacteria | 5.0 × 10⁵ cells/mL |
| Immobilizing material | 5 mass% |
| Polymerization accelerator | 0.1 mass% |
| Polymerization initiator | 0.05 mass% |
| Filler | 5 mass% |

### (Example 1) Shape and amount added of filler

Pellets produced in the same manner as above were examined for the relation between the shape of a filler added to an immobilizing material and the pellet strength. Table 2 shows results of measuring the relation between the shape of a filler added to 5 mass% of a polyethylene glycol prepolymer and the pellet strength. In the present embodiment, talc was used as a plate-like filler, and silica was used as a spherical filler.

**Table 2**

| [Unit: kgf/cm²] | | | |
|---|---|---|---|
| Ratio of filler added | Plate-like filler | Spherical filler | Filler not added (Blank) |
| 1 mass% | 4.5 (44.1) | 3.1 (30.4) | 2.74 (26.9) |
| 3 mass% | 6.5 (63.7) | 3.5 (34.3) | 2.74 (26.9) |
| 5 mass% | 7.2 (70.6) | 4.5 (44.1) | 2.74 (26.9) |
| 10 mass% | 8.4 (82.3) | 6.1 (59.8) | 2.74 (26.9) |

| | | | |
|---|---|---|---|
| Values within parentheses are based on unit N/cm². | | | |

As shown in Table 2, it was confirmed that pellets with any amount of a filler added have pellet strength improved as compared with pellets with a filler not added. It was also found that pellets with an amount of a plate-like filler added have pellet strength about 1.5 times that of pellets with the same amount of a spherical filler added. From this it was confirmed that a plate-like filler is highly effective for improving pellet strength.

### (Example 2) Amount of filler added

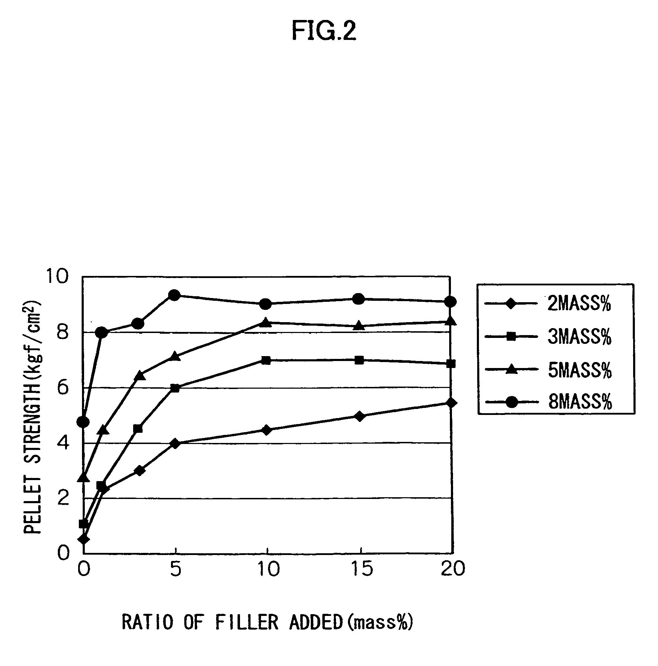

Next, the relation between the amount of a plate-like filler added to various concentrations of an immobilizing material and the pellet strength was examined. Fig. 2 shows results of measuring the pellet strength in the case where 1, 3, 5, 10, 15, or 20 mass% of a plate-like filler (talc) was added to 2, 3, 5, or 8 mass% of a polyethylene glycol prepolymer.

As shown in Fig. 2, when a plate-like filler was added in an amount of less than 10 mass%, the pellet strength was drastically increased as the amount of the filler was increased, but when the filler was added in an amount of 10 mass% or more, no significant change was observed. It was also found that, as the concentration of the immobilizing material is lower, the amount of the plate-like filler added more significantly affects the pellet strength.

As the amount of the plate-like filler added is increased, the specific gravity of the pellets is increased, and thus it is difficult to carry out complete mixing by aeration. For this reason, and taking the above results into consideration as well, the plate-like filler is suitably added in an amount of 3 to 10 mass%, although the amount may depend on other properties such as average particle size.

### (Example 3) Average particle size of filler

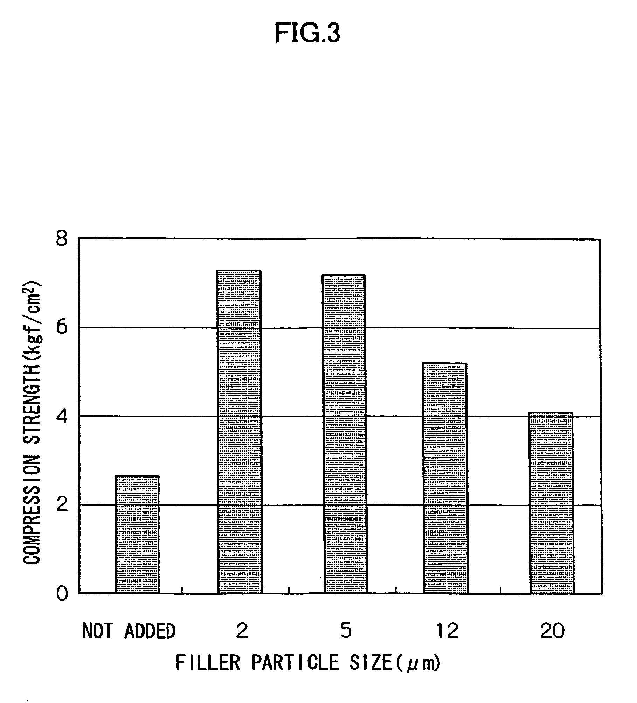

Further, the relation between the average particle size of a plate-like filler added to an immobilizing material and the pellet strength was examined. Fig. 3 shows results of measuring the relation between the average particle size of a plate-like filler (talc) and the pellet strength when the plate-like filler (talc) was added to 5 mass% of a polyethylene glycol prepolymer.

As shown in Fig. 3, the pellet strength was highest when the plate-like filler had an average particle size of about 12 µm or less, and the pellet strength tended to be decreased when the filler had an average particle size more than 12 µm. It was also confirmed that the pellet strength in the case where the plate-like filler had an average particle size of about 20 µm was higher than in the case where the filler was not added. From this, it was found that the plate-like filler has an average particle size of preferably 12 µm or less, and more preferably 10 µm or less.

### (Example 4) Evaluation of continuous nitrification performance

Continuous nitrification performance of the entrapping immobilization pellets of the present embodiment was evaluated.

### (Test conditions)

Test wastewater: Inorganic synthetic wastewater (ammonium nitrogen: about 40 mg/L)

Test pellets: The entrapping immobilization pellets of the present invention and conventional entrapping immobilization pellets were used. The compositions of the two types of pellets are shown in Table 3.

**Table 3**

| Composition | Inventive entrapping immobilization pellets | Conventional entrapping immobilization pellets |
|---|---|---|
| Activated sludge | 3 mass% | 3 mass% |
| Immobilizing material | 5 mass% | 15 mass% |
| Polymerization accelerator | 0.1 mass% | 0.5 mass% |
| Polymerization initiator | 0.05 mass% | 0.25 mass% |
| Filler | 5 mass% | - |

Treatment method: Aeration tanks b and c were respectively packed with the entrapping immobilization pellets of the present invention from different production lots at a packing ratio of 10% based on inorganic synthetic wastewater a. Aeration tanks d, e, and f were respectively packed with conventional entrapping immobilization pellets from different production lots at a packing ratio of 10% based on inorganic synthetic wastewater a. Inorganic synthetic wastewater a was allowed to flow into each of the aeration tanks b, c, d, e, and f and brought into contact with the entrapping immobilization pellets.

Treatment was carried out at a room temperature of 20°C, and the retention time in the aeration tanks was 3 hours. Ammonium nitrogen in the wastewater was measured by ion chromatography as a sewage testing method. The two types of entrapping immobilization pellets having different compositions were produced by the same process.

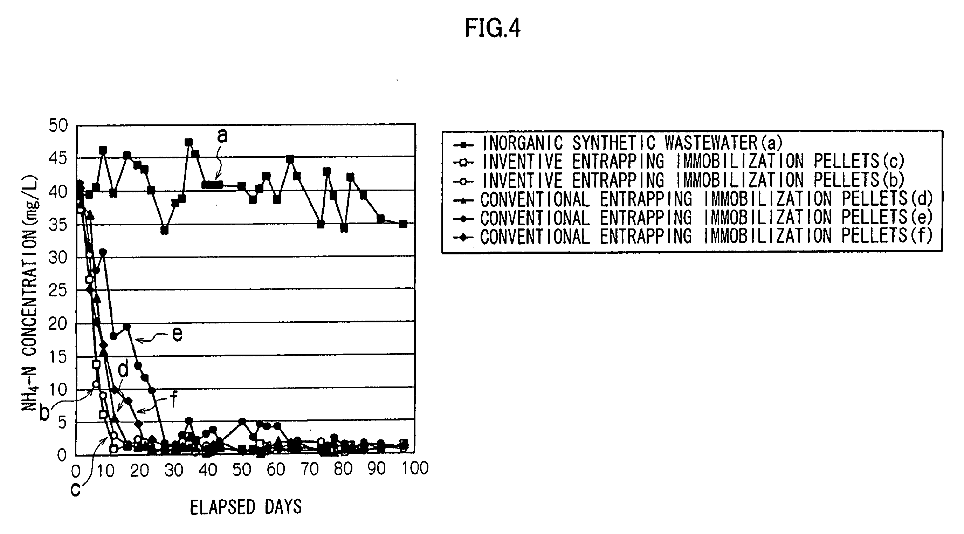

As shown in Fig. 4, it was confirmed that, when continuous operation was carried out for 100 days, the two kinds of the entrapping immobilization pellets of the present invention from different lots (b, c) drastically decreased the ammonium nitrogen concentration at the same time, and thereafter exhibited stable nitrification performance.

On the other hand, the three kinds of conventional entrapping immobilization pellets from different lots (d, e, f) exhibited the same stable nitrification performance after activity rising as in the entrapping immobilization pellets of the present invention, but the time needed for activity rising significantly differs according to the lots.

As is clear from the above results, the entrapping immobilization pellets of the present invention exhibit a smaller variation in nitrification performance according to the production lots as compared with conventional entrapping immobilization pellets, and have stable nitrification performance.

## Claims

1. A process for producing entrapping immobilization pellets in which microorganisms are entrapped and immobilized in an immobilizing material, the process comprising:
- mixing the immobilizing material with at least one plate-like and/or needle-like filler,
- mixing the mixture with the microorganisms to prepare a suspension,
- adding a polymerization initiator to said suspension and
- polymerizing the mixture to form a gel.

2. The process for producing entrapping immobilization pellets according to claim 1, wherein the plate-like and/or needle-like filler is an inorganic filler.

## Patentansprüche

1. Verfahren zur Herstellung von Pellets, die Mikroorganismen einschließen und immobilisieren, wobei die Mikroorganismen in einem Immobilisierungsmaterial eingeschlossen und immobilisiert sind, wobei das Verfahren folgende Stufen umfasst:
- Mischen des Immobilisierungsmaterials mit mindestens einem plattenförmigen und/oder nadelförmigen Füller,
- Mischen der Mischung mit den Mikroorganismen um eine Suspension zu formen,
- Zugabe eines Polymerisationsinitiatoren zu besagter Suspension, und
- Polymerisieren der Mischung um ein Gel zu formen.

2. Verfahren zur Herstellung von Pellets gemäß Anspruch 1, wobei der plattenförmige und/oder nadelförmige Füller ein anorganischer Füller ist.

## Revendications

1. Procédé pour produire des pastilles de piégeage et d'immobilisation dans lesquelles des micro-organismes sont piégés et immobilisés dans un matériau d'immobilisation, le procédé comprenant :
- le mélange du matériau d'immobilisation avec au moins une charge de type plaque et/ou de type aiguille,
- le mélange du mélange avec les micro-organismes pour préparer une suspension,
- l'addition d'un initiateur de polymérisation à ladite suspension et
- la polymérisation du mélange pour former un gel.

2. Procédé pour produire des pastilles de piégeage et d'immobilisation selon la revendication 1, où la charge de type plaque et/ou de type aiguille est une charge inorganique.
